# EUROPEAN PATENT APPLICATION

(11) **EP 1 243 181 A1**
(43) Date of publication of application: **25.09.2002**
(21) Application number: 01201086.4
(22) Date of filing: 23.03.2001
(51) Int. Cl.: A23C 9/123, A23C 19/06, A61K 35/74, A23K 1/00, C12R 1/46

(54) **A probiotic, a food product containing a probiotic, a method for preparation of said food product, a pharmaceutical composition and a use of the probiotic strain**

(71) Applicant: NIZO food research, 6718 ZB Ede (NL)
(72) Inventor: Bovee-Oudenhoven, Ingeborg Marie Jacqueline, 6604 GL Wijchen (NL); van der Meer, Roelof, 6712 HA Ede (NL)
(74) Representative: Kupecz, Arpad

(57) **Abstract**

The invention relates to a probiotic capable of exerting a protective effect in the gastro-intestinal tract. The probiotic is a deposited Lactococcus lactis strain (CBS108918, CBS109208) or a mutant or derivative thereof. The invention also relates to a food product, a poultry feed and a pharmaceutical composition containing the probiotic, as well as a method for the preparation thereof.

## Description

The present invention relates to a probiotic.

Probiotic-comprising food products are known for many years. A probiotic is a probiotic micro-organism is a micro-organism and in the context of the present invention it also encompasses a product excreted by a probiotic-organism, said probiotic contributing to the health and/or the well-being of a bird, such as poultry, or a mammal, such as a human. For example, probiotic-comprising food products are known having a beneficial effect when combating gastro-intestinal infections, such as infections with Rotavirus. The European patent application 0,904,784 discloses a food product comprising a mixture of probiotic strains, amongst which optionally a Lactococcus strain, for which Lactococcus lactis LW-P53 is mentioned as an example.

As probiotics are neither always effective with gastro-intestinal infections, nor capable of being included in any food product, there is a continuous need for new probiotics. In addition, in many cases a mixture of several probiotic strains is necessary for achieving an effect.

The present invention relates to a new probiotic which is characterized in that it is a probiotic micro-organism or a composition derived from such a micro-organism chosen from the group consisting of
- Lactococcus lactis strain B970 which was deposited on 27 June, 2000 with the Centraalbureau voor Schimmelcultures, (CBS, Oosterstraat 1, 3740 AG Baarn, the Netherlands) under accession number CBS 108918, and
- Lactococcus lactis strain B971 which was deposited on 20 November, 2000 with the CBS under accession number CBS 109208
- or a mutant or derivative thereof.

These Lactococcus strains are, surprisingly, capable to exert a protective effect, as shown by experiment, without the presence of further probiotic strains, something that has been disclosed up to now only for some Lactobacillus strains. In the present invention a probiotic micro-organism is understood to be a living micro-organism which may be in the form of spores, lyophilized form or otherwise non-lethally inactivated. Here, an inactivated probiotic micro-organism will be in an active form again before, during or after ingestion by a mammal or a bird in the gastro-intestinal tract, in which active state it may contribute to the health and/or the well-being of the animal, in particular a human being. In the present invention a mutant is understood to be any probiotic strain derived from the claimed strains, undergone a change in the nucleotide sequence in its chromosomal or extra-chromosomal DNA while having retained its probiotic quality. The change may be the result of a natural process, or an induction by various agents or by genetic engineering techniques. See for a detailed description and examples for instance J.H. Miller "A short course in bacterial genetics" Cold 1992, page 83 112. A derivative is understood to be any probiotic strain derived from the claimed strain that may be provided with extra DNA while having retained its probiotic quality. Mutants and derivatives may be produced using any technique for genetic engineering known in the art, for example as disclosed by Sambrook et al in Molecular Cloning - a laboratory manual, Cold Spring Harbor, New York, 1989.

The invention also relates to a food product containing the probiotic according to the invention.

In the present invention a food product is understood to be a food product for mammals, in particular for a human being, or animal feed, such as feed for poultry such as chicken.

According to a preferred embodiment the food product is a dairy product, preferably a dairy product chosen from buttermilk, yoghurt, cheeses and, in particular, soft cheeses, cottage cheese, curd and quark.

The probiotic according to the invention seems to be particularly suited for solid or semi-solid food products.

For non-human animals, a particular embodiment of the food product is a feed containing a probiotic according to the invention, in particular poultry feed.

The present invention also relates to a method for the preparation of a food product containing the probiotic according to the invention.

The method is characterized in that the probiotic micro-organism is grown and after an optional isolation in part or whole the micro-organism or an optionally purified probiotically active composition derived from said micro-organism is added to the food product, wherein after the addition the food product is not subjected to any probiotic micro-organism-killing or pro-biotic activity of the composition eliminating processes.

Thus a food product can be obtained having beneficial effects to a animal. The process or processes to which the food product is subjected after addition of the probiotic may comprise inactivation of the probiotic micro-organism according to the invention as long the micro-organism is not killed. Typically the food product according to the invention comprises more than 10⁶ probiotic micro-organism/g food product, such as at least 10⁷/g. The probiotic micro-organism may be grown on the food product, a half-finished product or starting material, or may be added at any stage during the preparation of the food product. Addition occurs, depending on what is desired, in the presence of substrate on which the probiotic micro-organism was grown or after separation from the probiotic micro-organism from the substrate.

The present invention also relates to a pharmaceutical composition containing the probiotic according to the invention, together with a pharmaceutically acceptable carrier or excipient.

Such a composition may be used for curative or prophylactic purposes in order to improve or maintain the health and/or the well-being of a mammal, in particular a human being. Similarly, it may be used to reduce the occurrence of Salmonella spp. in poultry. That is, it may reduce the number of Salmonella bacteria per chicken, as well reduce the number of chicken carrying Salmonella.

Finally the invention relates to the use of a probiotic according to the invention for the preparation of pharmaceutical composition, suitable for the oral application against infections and/or food poisoning caused by Gram-negative micro-organisms.

The importance is not the least the fact that more and more micro-organisms become resistant against antibiotics. The claimed use for poultry and mammals, in particular a human being, may eliminate (prevent), replace or supplement treatment with antibiotics.

The present invention will be elucidated with reference to the following examples and the drawing in which the only figure graphically depicts the intestinal colonization of Salmonella as measured by the faecal excretion of that pathogen in time.

### 1. Animal experiments

Specific pathogen-free mail Wistar rats (Wu, Harlan, Horst, the Netherlands), age 8 weeks having a mean body weight of 263 g, were individually housed in cages in a room with controlled temperature (22-24°C), relative humidity (50-60%) and a light/dark cycle (light from 6 a.m. to 6 p.m.). The animals were randomly divided in 4 groups of 8 animals each. For the entire period of the experiment, the animals had free excess to demineralized drinking water and food. Every 2 days the food consumption was measured and the body weight was measured every day.

After 4 days the following probiotics were orally administered daily:
- Lactobacillus rhamnosus GG, a probiotic known from a dairy product currently on the market, or
- Lactococcus lactis B970 according to the invention, or
- Lactococcus lactis B971 according to the invention (isolated from saliva of a cow) .

The lactic acid bacteria were suspended in 0.5 ml fresh sterile MRS broth (Merck, Darmstadt, Germany). A control group received only 0.5 ml fresh MRS broth. The daily doses of lactic acid bacteria was about 10¹⁰ CFU, as determined by growing on agar plates. The probiotic suspensions were freshly prepared daily by thawing of a frozen (-80°C) vial containing 1% of a stationary culture (suspended in litmus-containing skimmed milk with 0.5% yeast extract) on a water bath at room temperature. The medium was inocculated with 2% of a thawed suspension of bacteria. In particular this comprised:
- MRS medium supplemented with 0.05% cystein for Lb. Rhamnosus GG; and
- M17 medium (Oxoid, Basingstoke, England) supplemented with 0.5% lactose for Lactococcus lactis B970 and B971.

Lactobacilli were grown under an atmosphere of 80% nitrogen, 10% carbon dioxide and 10% hydrogen at 37°C for 24 h. The Lactococci were aerobically grown for 24 h. at 37°C. The lactic acid bacteria were harvested by means of centrifugation (20 min. at 2,000 g; tabletop centrifuge) and resuspended in fresh sterile MRS medium and administered to the animals.

Salmonella entiritidis was grown as described (ref. 2 and 3) for infection of the animals. After 11 days of treatment with probiotics the animals were infected by oral administration of a 0.5 ml saline comprising 3% sodium bicarbonate and 3 x 10⁸ SFU S. enteritidis. Before and several days after infection fresh faecal samples were obtained directly from the anus of the animals. The number of Samonella bacteria in the faeces were quantified by plating 10-fold dilutions of faeces on modified Brilliant Green Agar (Oxoid) containing sulphamandelate (ref. 4). The experiment was ended 10 days after infection.

### Results:

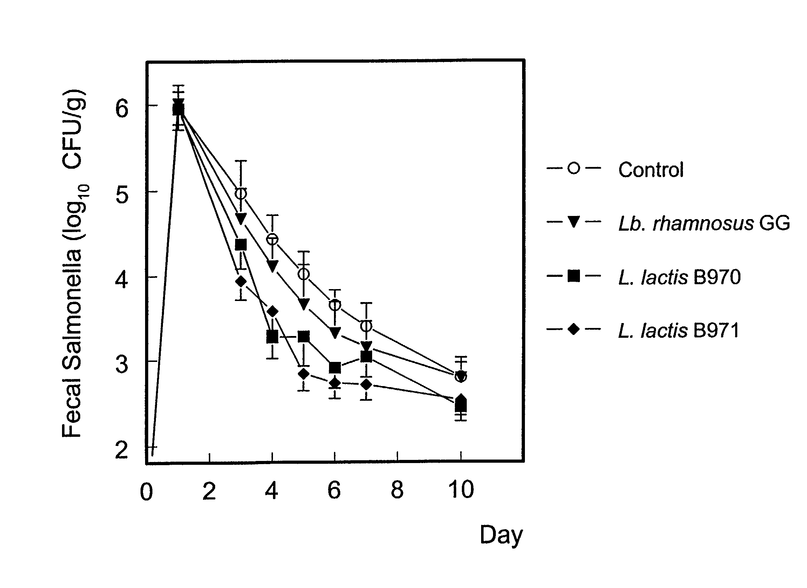

The food uptake by and growth of the animals were neither affected by the treatment with probiotics nor by the infection. The average daily gain in body weight was 3 g and the intake of food was 21 g per day (as dry weight). The Lactococci according to the invention improved the resistance to colonization of the rats against S. enteriditis significantly, as shown by the reduced faecal excretion of this pathogen in time (Fig. 1. Please note the logarithmic scale). There was a significantly reduced colonization on the days 4-6 after infection of the rats. In contrast, Lactobacillus rhamnosus GG did not protect against S. enteritidis infection as the faecal excretion of this pathogen was not significantly different from the sham-treated control group.

### REFERENCES

1. Reeves et al. J. Nutr. 123, pp. 1939-1951 (1993).
2. Bovee-Oudenhoven, I.M.J. et al. Gut 40, pp. 497-504 (1997).
3. Oudenhoven, I.M.J. et al. Gastroenterol. 107, pp. 47-53 (1994).
4. Bartram, H.P. et al. Am. J. Clin. Nutr. 59: pp. 428-432 (1994).

## Claims

1. Probiotic **characterized in that** it is a probiotic micro-organism or a composition derived from such a micro-organism chosen from the group consisting of
- Lactococcus lactis strain B970 which was deposited on 27 June, 2000 with the Centraalbureau voor Schimmelcultures, (CBS, Oosterstraat 1, 3740 AG Baarn, the Netherlands) under accession number CBS 108918, and
- Lactococcus lactis strain B971 which was deposited on 20 November, 2000 with the CBS under accession number CBS 109208
- or a mutant or derivative thereof.

2. Food product containing a probiotic, **characterized, in that** the food product contains Lactococcus lactis strain with accession number CBS 108918 and/or number CBS 109208 as a probiotic.

3. Food product according to claim 2, **characterized, in that** the food product is a dairy product.

4. Food product according to claim 3, **characterized, in that** the dairy product is chosen from buttermilk, yoghurt, cheeses, and in particular soft cheeses, cottage cheese, curd and quark.

5. Food product according to claim 2, **characterized in that** the food product is poultry feed.

6. Method for the preparation of a food product containing a probiotic, **characterized, in that** the probiotic micro-organism is grown and after an optional isolation in part or whole the micro-organism or an optionally purified probiotically active composition derived from said micro-organism is added to the food product, wherein after the addition the food product is not subjected to any probiotic micro-organism-killing or pro-biotic activity of the composition eliminating processes.

7. Pharmaceutical composition containing the strain according to claim 1, together with a pharmaceutically acceptable carrier or excipient.

8. Use of a probiotic according to claim 1, for the preparation of a pharmaceutical composition, suitable for oral administration against infections and/or food poisoning by Gram-negative micro-organisms.
